# EUROPÄISCHE PATENTSCHRIFT

(11) **EP 1 078 330 B1**
(45) Veröffentlichungstag und Bekanntmachung des Hinweises auf die Patenterteilung: **07.08.2002**
(21) Anmeldenummer: 99936259.3
(22) Anmeldetag: 17.05.1999
(51) Int. Cl.: G06K 9/00, G06K 11/16, G06K 9/20, A61B 5/117

(54) **SENSOREINRICHTUNG ZUR ERFASSUNG VON BIOMETRISCHEN MERKMALEN, INSBESONDERE FINGERMINUTIEN**
SENSOR DEVICE FOR DETECTING BIOMETRIC CHARACTERISTICS, ESPECIALLY FINGERPRINT MINUTIAE
SYSTEME DE DETECTION DE CARACTERISTIQUES BIOMETRIQUES, NOTAMMENT D'EMPREINTES DIGITALES

(30) Priorität: 19.05.1998 DE 19822504
(43) Veröffentlichungstag der Anmeldung: 28.02.2001
(73) Patentinhaber: Infineon Technologies AG, 81669 München (DE)
(72) Erfinder: FRIES, Manfred, D-94336 Hunderdorf (DE); FISCHBACH, Reinhard, D-93049 Regensburg (DE); HOUDEAU, Detlef, D-84085 Langquaid (DE)
(74) Vertreter: Epping Hermann & Fischer
(86) Internationale Anmeldenummer: DE9901478
(87) Internationale Veröffentlichungsnummer: WO9960513

(56) Entgegenhaltungen:
- EP-A- 0 786 745
- WO-A-98/11500
- FR-A- 2 736 179

## Beschreibung

Die Erfindung betrifft eine Sensoreinrichtung zur Erfassung von biometrischen Merkmalen, insbesondere Fingerminutien, mittels eines biometrischen Sensorchips, wobei der Sensorchip an einer Flexleiterplatte befestigt ist, die aus einer hochflexiblen Trägerschicht und auf der Trägerschicht aufgebrachten Leiterbahnen besteht, die mit dem Sensorchip in elektrischen Kontakt stehen und zu einem Anschlußbereich der Flexleiterplatte geführt sind.

Es ist bekannt, personenspezifische Merkmale, beispielsweise Fingerminutien, d.h. Fingerabdrücke, mittels biometrischer Fingertippsensoren zu erfassen, um in Abhängigkeit des Erfassungsergebnisses den Zugang zu einem Gerät, einem Raum etc. zu ermöglichen oder zu verweigern. Eine derartige Authentifizierung von Personen mittels biometrischer Daten kann beispielsweise bei Bankautomaten, Handys und Computer eingesetzt werden.

Bekannte Sensoreinrichtungen dieser Art werden üblicherweise dadurch hergestellt, daß der Sensorchip auf eine Trägerplatte aufgesetzt wird, daß anschließend die Anschlußpads des Sensorchips mit den Leiterbahnen auf der Trägerplatte mittels eines Wirebonding-Verfahrens verbunden werden und der Sensorchip mit einer Masse eingekapselt wird, um ihn an der Trägerplatte stabil zu halten und zu schützen. Eine derartige Anordnung ist beispielsweise aus der FR 2 736 179 A1 bekannt. Nachteilig ist hierbei jedoch, daß eine derartige Anordnung einen relativ aufwendigen Herstellprozeß erfordert. Weiterhin ist oftmals die Montage einer derartigen Sensoreinrichtung in dem aufnehmenden Gehäuse relativ kompliziert und toleranzkritisch.

Eine andere bekannte Sensoreinrichtung ist aus der EP 0 786 745 A2 bekannt. Dort ist ein den Sensorchip umgebendes Gehäuse aus einer Plastikvergußmasse derart ausgebildet, daß die Sensorfläche über eine Öffnung im Gehäuse zugänglich ist. Weiterhin beschreibt die WO 98/11500 eine Sensoreinrichtung, bei der der Sensor mit dünnen Metall-Layern um einen flexiblen Plastikträger herum aufgebaut ist. Leiterbahnen auf dem Plastikträger stellen eine Verbindung zwischen dem Sensor und einem Anschlußbereich der flexiblen Plastikträger her. Der Aufbau des in der WO 98/11500 beschriebenen Sensoreinrichtung ist jedoch kompliziert und schwierig in unterschiedliche Geräte einzubauen.

Der Erfindung liegt daher die Aufgabe zugrunde, eine Sensoreinrichtung der eingangs genannten Art zu schaffen, die besonders einfach herstellbar ist und darüberhinaus auf einfache Weise in Geräte eingebaut werden kann.

Diese Aufgabe wird erfindungsgemäß durch die Merkmale des Anspruchs 1 gelöst. Vorteilhafte Ausführungsformen der Erfindung sind in den weiteren Ansprüchen beschrieben.

Bei der erfindungsgemäßen Sensoreinrichtung ist der Sensorchip an einer Flexleiterplatte befestigt, die aus einem hochflexiblen Trägermaterial und auf dem Trägermaterial aufgebrachten Leiterbahnen besteht, die mit dem Sensorchip in elektrischem Kontakt und zu einem Anschlußbereich der Flexleiterplatte geführt sind.

Die erfindungsgemäße Sensoreinrichtung bietet den Vorteil, daß die Sensorchips auf Flexleiterplatten montiert und getestet werden können, die in Form von Endlosbändern oder Nutzen, d.h. größeren Bogen, auf denen sich eine Mehrzahl von Flexleiterplatten befinden, vorliegen. Der Einbau der Sensoreinrichtungen in die Geräte ist in den Herstellprozeß leicht zu integrieren und dementsprechend kostengünstig. Die Verbindung zwischen dem Anschlußbereich der Flexleiterplatte zum Gerät kann über eine Standardsteckverbindung, beispielsweise einen Nullkraftstecker, oder eine Lötverbindung erfolgen. Weiterhin kann die Flexleiterplatte entsprechend den Kundenwünschen konfektioniert werden, d.h. die Länge, Breite, Form der Anschlüsse etc. der Flexleiterplatte kann auf einfache Weise individuell ausgelegt werden. Aufgrund der Flexibilität der Flexleiterplatte ist die Sensoreinrichtung darüberhinaus auch bei größeren Höhentoleranzen des Geräteaufnahmeraums leicht zu montieren.

Der Sensorchip ist dabei an der Flexleiterplatte derart befestigt, daß das Sensorfeld des Sensorchips durch eine Durchgangsöffnung der Flexleiterplatte hindurch zugänglich ist. Die Durchgangsöffnung der Flexleiterplatte ist auf der Berührungsseite zumindest teilweise von einem elektrisch leitenden Masserahmen umgeben, der mit einer Leiterbahn der Flexleiterplatte in elektrisch leitender Verbindung steht. Ein derartiger Masserahmen wird beim Auflegen beispielsweise eines Fingers auf die Sensoreinrichtung zwangsläufig vom Finger kontaktiert und leitet Spannungsspitzen vom Finger zur Erde ab. Ein derartiger Masserahmen kann sehr einfach und auf die gleiche Weise wie die Leiterbahnen auf das flexible Trägermaterial der Flexleiterplatte aufgebracht werden.

Gemäß einer vorteilhaften Ausführungsform weist die Sensoreinrichtung ein formstabiles Halte- und Führungsteil für den Sensorchip mit einer Vertiefung auf, in die der Sensorchip einsetzbar und in welcher der Sensorchip festlegbar ist. Ein derartiges Halte- und Führungsteil dient zur Befestigung der gesamten Sensoreinrichtung am Gerät, positioniert den Sensorchip mittels der Vertiefung und schützt zugleich den Sensorchip, da dieser in der Vertiefung eingebettet ist.

Da der Sensorchip von der Unterseite der Flexleiterplatte her an dieser befestigt wird, ist es zweckmäßig, wenn die Sensor-Pads, d.h. seine Anschlußkontakte, an der Oberseite des Sensorchips angeordnet sind, so daß auf der Unterseite der Flexleiterbahn angeordnete Leiterbahnen direkt auf die Sensorchip-Pads aufgelegt und mit diesen elektrisch verbunden werden können. Diese elektrische Verbindung kann durch bekannte Verfahren wie Heißsiegeln, Löten, Thermokompression, Thermosonic, Wirebonden usw. durchgeführt werden. Es ist jedoch auch ohne weiteres möglich, die Leiterbahnen der Flexleiterplatte zusätzlich oder alternativ auf der Oberseite der Trägerschicht aufzubringen und durch einen Durchbruch in der Trägerschicht hindurch und den Sensor-Pads zu kontaktieren.

Die Erfindung wird nachfolgend anhand der Zeichnungen beispielshaft näher erläutert. In diesen zeigen:
- Figur 1 :: eine Explosionsdarstellung der erfindungsgemäßen Sensoreinrichtung mit Flexleiterplatte, Sensor-chip und Halte- und Führungsteil,
- Figur 2 :: einen Längsschnitt durch die Sensoreinrichtung von Figur 1 vor dem Einsetzen des Sensorchips in das Halte- und Führungsteil,
- Figur 3 :: eine Darstellung entsprechend Figur 2 nach dem
- Figur 4: einen Teil eines Längsschnitts längs der Linie IV- IV von Figur 6 bei montiertem Sensorchip,
- Figur 5 :: einen Teil eines Längsschnitts längs der Linie V-V von Figur 6 bei montiertem Sensorchip, und
- Figur 6 :: eine Draufsicht auf die Flexleiterplatte.

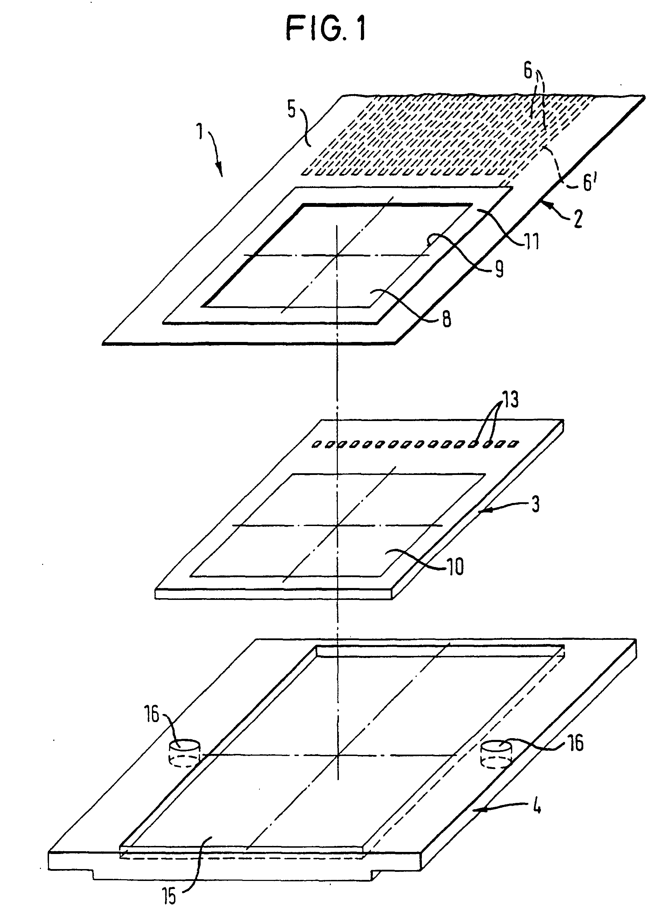

Die in Figur 1 explosionsartig dargestellte Sensoreinrichtung 1 besteht im wesentlichen aus einer Flexleiterplatte 2, einem Sensorchip 3 und einem Halte- und Führungsteil 4.

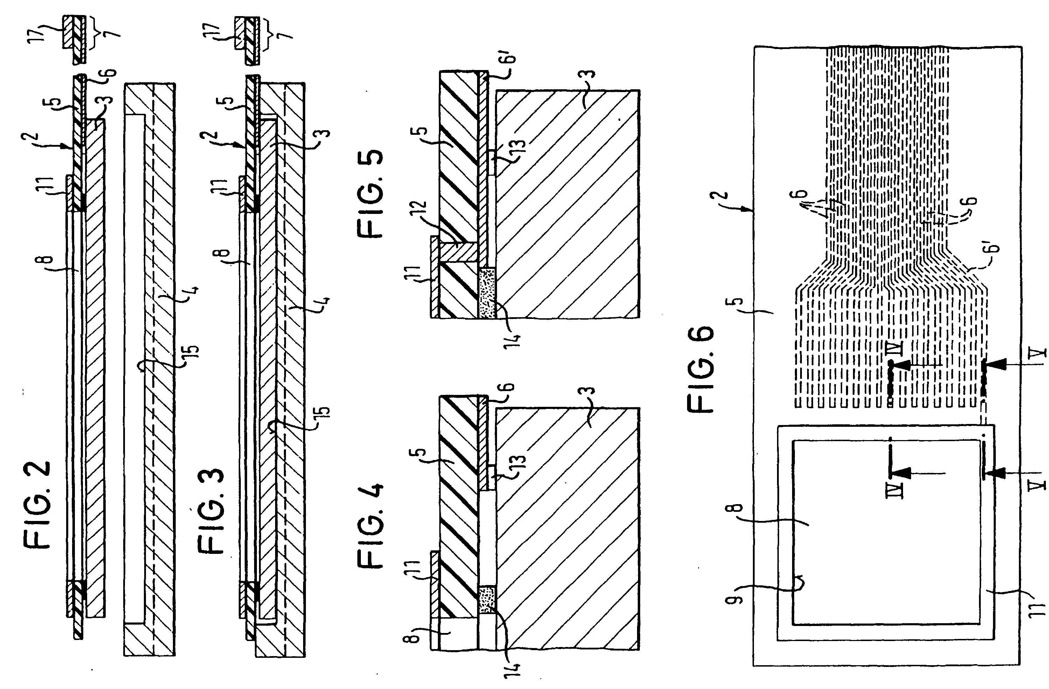

Die Flexleiterplatte 2 besteht aus einer dünnen, hochflexiblen, nicht leitenden Trägerschicht 5, beispielsweise aus Kapton oder einer PET-Folie. Auf der Unterseite dieser Trägerschicht 5 sind eine Vielzahl von Leiterbahnen 6 aufgebracht, die sich bei dem dargestellten Ausführungsbeispiel im wesentlichen in Längsrichtung der streifenartigen Flexleiterplatte 2 von einem Anschlußbereich 7, der sich an einem Ende der Flexleiterplatte 2 befindet, in Richtung einer rechteckigen oder quadratischen Durchgangsöffnung 8 erstrecken. Der Rand dieser Durchgangsöffnung 8 ist in den Figuren 1 und 6 mit dem Bezugszeichen 9 versehen. Die Größe der Durchgangsöffnung 8 entspricht in etwa der Größe eines Sensorfeldes 10 des Sensorchips 3. Als Sensorfeld 10 wird hierbei diejenige sensitive Fläche des Sensorchips 3 bezeichnet, welche die Minutien eines auf das Sensorfeld 10 aufgelegten Fingers erfassen kann.

Auf der Oberseite der Flexleiterplatte 2, d.h. auf der den Leiterbahnen 6 gegenüberliegenden Seite der Trägerschicht 5, ist ein Masserahmen 11 auf die Trägerschicht 5 aufgebracht, der die Durchgangsöffnung 8 im Bereich des Randes 9 vollständig umgibt. Dieser Masserahmen 11 besteht aus einem elektrisch leitenden Material, so daß bei Kontakt mit einem Finger dort vorhandene Spannungsspitzen abgeleitet werden können. Zu diesem Zweck ist der Masserahmen 11 über eine die Trägerschicht 5 durchsetzende Durchkontaktierung 12 (Figur 5) mit einer Masseleiterbahn 6' elektrisch verbunden, die sich in gleicher Weise wie die Leiterbahnen 6 auf der Unterseite der Flexleiterplatte 2 befindet.

Die Leiterbahnen 6, die Masseleiterbahn 6' und der Masserahmen 11 werden dadurch hergestellt, daß auf die Trägerschicht 5 eine Kupferfolie oder Silberleitpaste aufgebracht wird. Anschließend werden sie durch Ätzen geeignet strukturiert und mit einer geeigneten Metallisierung, z.B. aus SnPb oder NiAu, versehen, um eine Oxidierung zu verhindern.

Wie weiterhin aus Figur 6 ersichtlich ist, erstrecken sich die Leiterbahnen 6 in Längsrichtung nicht ganz bis zum Masserahmen 11, sondern enden kurz vor diesem. Die Masseleiterbahn 6' erstreckt sich dagegen in Längsrichtung bis unter den Masserahmen 11, so daß eine senkrechte Durchkontaktierung 12 genügt, um die elektrische Verbindung zwischen dem Masserahmen 11 und der Masseleiterbahn 6' herzustellen.

Der Sensorchip 3 weist an seiner Oberseite freiliegende Pads 13 (Anschlußkontakte) auf. Diese Pads 13 sind mit einem gewissen Abstand vor dem Sensorfeld 10 derart angeordnet, daß jedes Pad 13 mit einer zugeordneten Leiterbahn 6 in Kontakt ist, wenn der Sensorchip 3 von unten in der vorbestimmten Weise an der Flexleiterplatte 2 befestigt ist. Die Festlegung des Sensorchips 3 an der Flexleiterplatte 2 erfolgt mittels eines Klebers 14, der benachbart zum Rand 9 der Durchgangsöffnung 8 aufgebracht wird. Der Sensorchip 3 ist hierbei derart an der Flexleiterplatte 2 montiert, daß das Sensorfeld 10 zur Durchgangsöffnung 8 ausgerichtet ist. Das Sensorfeld 10 zeigt im montierten Zustand nach oben, so daß es durch die Durchgangsöffnung 8 hindurch mit dem Finger kontaktiert werden kann.

Der an der Flexleiterplatte 2 montierte Sensorchip 3 wird anschließend in eine Vertiefung 15 des Halte- und Führungsteils 4 eingesetzt und darin beispielsweise durch Verkleben festgelegt. Die Vertiefung 15 ist an die Außenkontur des Sensorchips 3 derart angepaßt, daß der Sensorchip 3 nur mit geringem Spielraum in die Vertiefung 15 eingesetzt wird, so daß eine exakte Führung und Positionierung für den Sensorchip 3 gewährleistet ist. Die Tiefe der Vertiefung 15 ist derart bemessen, daß der Sensorchip 3 im wesentlichen vollständig eingebettet ist, d.h. die Oberfläche des Sensorchips 3 fluchtet im eingesetzten Zustand mit der seitlich angrenzenden Oberfläche des Halte- und Führungsteils 4. Die mechanische Stabilität des Sensorchips 3 wird somit einerseits durch eine relativ große Chipdicke und andererseits durch den Einbau des Sensorchips 3 in das Halte- und Führungsteil 4 erreicht, das aus einem entsprechend biegefesten Material besteht.

Das plattenartige Halte- und Führungsteil 4 weist ferner in den Seitenbereichen vertikale Löcher 16 auf, die entweder als Schraubenlöcher zum Befestigen der Sensoreinrichtung 1 an einem Gehäuse oder als Positionierungshilfen dienen, um die Sensoreinrichtung 1 auf entsprechend vorstehende Dome des Gehäuses aufstecken zu können.

Alternativ zu der beschriebenen Ausführungsform ist es ohne weiteres möglich, die Leiterbahnen 6 zusätzlich oder alternativ auf der Oberseite der Trägerschicht 5 auszubilden. In diesem Fall weist die Trägerschicht 5 entsprechende Durchbrüche auf, um die Pads 13 beispielsweise mittels des Wirebonding-Verfahrens mit den auf der Oberseite der Trägerschicht 5 befindlichen Leiterbahnen verbinden zu können.

Die Leiterbahnen 6 und die Masseleiterbahn 6' können im Bereich des Anschlußbereichs 7 in einer nicht näher dargestellten Standardsteckverbindung, beispielsweise einem Nullkraftstecker, enden. In diesem Bereich ist die Flexleiterplatte 2 durch eine Querverstärkungsleiste 17 gegen eine zu leichte mechanische Durchbiegung gesichert. Anstelle einer Steckverbindung können in diesem Endbereich auch ohne weiteres entsprechende Lötverbindungen vorgenommen werden.

Die Länge der Flexleiterplatte 2 ist in den Figuren 1 bis 6 nur verkürzt dargestellt. Wie erkennbar, kann die Länge der Flexleiterplatte 2 auf einfache Weise den individuellen Einbauerfordernissen und Kundenwünschen angepaßt werden. Beispielsweise kann die Länge der Flexleiterplatte 2 das 1,5 bis zig-fache der Länge des Sensorchips 3 betragen.

Es ist zu beachten, daß die Abmessungen, insbesondere die Dickenverhältnisse, aus Gründen einer klareren Darstellung nicht maßstabsgetreu dargestellt sind.

## Patentansprüche

1. Sensoreinrichtung (1) zur Erfassung von biometrischen Merkmalen, insbesondere Fingerminutien, mittels eines biometrischen Sensorchips (3), wobei der Sensorchip (3) an einer Flexleiterplatte (2) befestigt ist, die aus einer hochflexiblen Trägerschicht (5) und auf der Trägerschicht (5) aufgebrachten Leiterbahnen (6, 6') besteht, die mit dem Sensor-chip (3) in elektrischem Kontakt stehen und zu einem Anschlußbereich (7) der Flexleiterplatte (2) geführt sind,
**dadurch gekennzeichnet, daß** der Sensorchip (3) an der Flexleiterplatte (2) derart befestigt ist, daß das Sensorfeld (10) des Sensorchips (3) durch eine Durchgangsöffnung (8) der Flexleiterplatte (2) hindurch zugänglich ist und die Durchgangsöffnung (8) der Flexleiterplatte (2) auf der Berührungsseite zumindest teilweise von einem elektrisch leitenden Masserahmen (11) umgeben ist, der mit einer Masseleiterbahn (6') der Flexleiterplatte (2) in elektrisch leitender Verbindung steht.

2. Sensoreinrichtung nach Anspruch 1,
**dadurch gekennzeichnet, daß** die Sensoreinrichtung (1) ein formstabiles Halte- und Führungsteil (4) für den Sensorchip (3) mit einer Vertiefung (15) aufweist, in die der Sensorchip (3) einsetzbar und in welcher der Sensorchip (3) festlegbar ist.

3. Sensoreinrichtung nach Anspruch 1 oder 2,
**dadurch gekennzeichnet, daß** die Leiterbahnen (6, 6') auf der Unterseite der Flexleiterplatte (2) angeordnet und mit Sensorchip-Pads (13) elektrisch verbunden sind, die auf der Oberseite des Sensorchips (3) angeordnet sind.

4. Sensoreinrichtung nach einem der vorhergehenden Ansprüche,
**dadurch gekennzeichnet, daß** die Trägerschicht (5) der Flexleiterplatte (2) aus Kapton oder einer PET-Folie besteht.

## Claims

1. Sensor device (1) for sensing biometric characteristics, in particular finger minutiae, by means of a biometric sensor chip (3), the sensor chip (3) being fastened on a flexible printed circuit board (2) which comprises a highly flexible substrate layer (5) and conductor tracks (6, 6') which have been applied to the substrate layer (5), are in electrical contact with the sensor chip (3) and are led to a terminal region (7) of the flexible printed circuit board (2), **characterized in that** the sensor chip (3) is fastened on the flexible printed circuit board (2) in such a way that the sensor zone (10) of the sensor chip (3) is accessible through a through-opening (8) in the flexible printed circuit board (2) and the through-opening (8) in the flexible printed circuit board (2) is surrounded on the contact side at least partially by an electrically conducting grounding frame (11), which is in electrically conducting connection with a grounding conductor track (6') of the flexible printed circuit board (2).

2. Sensor device according to Claim 1, **characterized in that** the sensor device (1) has a dimensionally stable holding and guiding part (4) for the sensor chip (3) with a depression (15), into which the sensor chip (2) can be placed and in which Sensor chip (3) can be fixed.

3. Sensor device according to Claim 1 or 2, **characterized in that** the conductor tracks (6, 6') are arranged on the underside of the flexible printed circuit board (2) and are electrically connected to sensor chip pads (13), which are arranged on the upper side of the sensor chip (3).

4. Sensor device according to one of the preceding claims, **characterized in that** the substrate layer (5) of the flexible printed circuit board (2) consists of Kapton or a PET film.

## Revendications

1. Dispositif (1) de détection de caractéristiques biométriques, notamment d'empreintes digitales, au moyen d'une puce (3) biométrique formant capteur, la puce (3) formant capteur étant fixée sur une plaquette (2) de circuit imprimé souple, qui est constituée d'une couche (5) support très souple et de pistes (6,6') conductrices déposées sur la couche (5) de support qui ont un contact électrique avec la puce (3) formant capteur et qui vont vers une partie (7) de connexion de la plaquette (2) à circuit imprimé souple, **caractérisé en ce que** la puce (3) formant capteur est fixée à la plaquette (2) à circuit imprimé souple de façon que le champ (10) de capteur de la puce (3) formant capteur soit accessible par une ouverture (8) de traversée de la plaquette (2) à circuit imprimé souple et l'ouverture (8) de traversée de la plaquette (2) à circuit imprimé souple est entourée, du côté que l'on peut toucher, au moins en partie d'un cadre (11) à la masse, conducteur de l'électricité, qui est en liaison conductrice de l'électricité avec une piste (6') conductrice à la masse de la plaquette (2) à circuit imprimé souple.

2. Dispositif suivant la revendication 1, **caractérisé en ce que** le dispositif (1) comporte une partie (4) de forme stable de maintien et de guidage de la puce (3) formant capteur et ayant une cavité (15) dans laquelle la puce (3) formant capteur peut être mise et dans laquelle la puce (3) formant capteur peut être fixée.

3. Dispositif suivant la revendication 1 ou 2, **caractérisé en ce que** les pistes (6,6') conductrices sont disposées sur la face inférieure de la plaquette (2) à circuit imprimé souple et sont reliées électriquement à des plages (13) de connexion de la puce (3) formant capteur, plages qui sont disposées sur la face supérieure de la puce (3) formant capteur.

4. Dispositif suivant l'une des revendications précédentes, **caractérisé en ce que** la couche (5) de support de la plaquette (2) à circuit imprimé souple est en kapton ou en feuille de PET.
